# EUROPEAN PATENT APPLICATION

(11) **EP 3 432 347 A1**
(43) Date of publication of application: **23.01.2019**
(21) Application number: 17766545.2
(22) Date of filing: 09.03.2017
(51) Int. Cl.: H01L 21/3065, C07C 19/08

(54) **DRY-ETCHING COMPOSITION AND CONTAINER FILLED WITH DRY-ETCHING COMPOSITION**

(30) Priority: 15.03.2016 JP 2016050790
(71) Applicant: Zeon Corporation, Tokyo 100-8246 (JP)
(72) Inventor: HYAKUTAKE Munehiro, Tokyo 100-8246 (JP)
(74) Representative: Adam, Holger
(86) International application number: PCT/JP2017/009570
(87) International publication number: WO 2017/159544

(57) **Abstract**

The objective is to inhibit fluorinated saturated hydrocarbon decomposition during storage or use of a dry etching composition containing a fluorinated saturated hydrocarbon in high concentration. A dry etching composition contains 99 volume% or more of a fluorinated saturated hydrocarbon and further contains an amine compound. The dry etching composition is preferably an azeotropic composition or an azeotrope-like composition.

## Description

### TECHNICAL FIELD

The present disclosure relates to a dry etching composition and a dry etching composition-filled container.

### BACKGROUND

Compositions containing fluorinated saturated hydrocarbons such as 2-fluorobutane in high concentration are conventionally used as dry etching gases (for example, refer to PTL 1).

### CITATION LIST

### Patent Literature

PTL 1: WO 2014/136877 A1

### SUMMARY

### (Technical Problem)

However, when a composition contains a fluorinated saturated hydrocarbon in a high concentration of 99 volume% or more, for example, there are cases in which the fluorinated saturated hydrocarbon decomposes during storage or use, thereby lowering the concentration of the fluorinated saturated hydrocarbon. In particular, fluorinated saturated hydrocarbon decomposition readily occurs when a composition containing a fluorinated saturated hydrocarbon in high concentration comes into contact with a metal such as stainless steel or manganese steel, or a solid acid such as aluminum oxide, silicon dioxide, a zeolite, or a cation exchange resin (proton type).

There has been demand, therefore, to inhibit fluorinated saturated hydrocarbon decomposition during storage or use of a composition containing a fluorinated saturated hydrocarbon in high concentration.

### (Solution to Problem)

In view of the above, the inventor conducted diligent investigation with the aim of inhibiting fluorinated saturated hydrocarbon decomposition. Through this investigation, the inventor reached a new discovery that by compounding an amine compound in a composition that contains a fluorinated saturated hydrocarbon in high concentration, decomposition of the fluorinated saturated hydrocarbon over time can be inhibited. In this manner, the inventor completed the present disclosure.

Specifically, the present disclosure aims to advantageously solve the problems set forth above by disclosing a dry etching composition comprising: 99 volume% or more of a fluorinated saturated hydrocarbon; and an amine compound. Through inclusion of an amine compound in a composition containing a fluorinated saturated hydrocarbon in high concentration in this manner, decomposition of the fluorinated saturated hydrocarbon can be inhibited.

The concentration of a fluorinated saturated hydrocarbon referred to in the present disclosure can be measured using a gas chromatograph.

The presently disclosed dry etching composition is preferably an azeotropic composition or an azeotrope-like composition. In a case in which the dry etching composition is an azeotropic composition or an azeotrope-like composition, the fluorinated saturated hydrocarbon and the amine compound are both favorably vaporized when the dry etching composition is vaporized for use, and this can inhibit variation of the make-up of the dry etching composition during use.

The term "azeotropic composition" as used in the present disclosure refers to a mixture for which a gas phase in equilibrium with a liquid phase has the same make-up as the liquid phase, whereas the term "azeotrope-like composition" as used in the present disclosure refers to a mixture for which a gas phase in equilibrium with a liquid phase has a similar make-up to the liquid phase.

In the presently disclosed dry etching composition, an absolute value of a difference between a boiling point of the fluorinated saturated hydrocarbon and a boiling point of the amine compound is preferably 25°C or less. When the absolute value of the difference between the boiling point of the fluorinated saturated hydrocarbon and the boiling point of the amine compound is 25°C or less, variation of the make-up of the dry etching composition during use thereof in a gaseous state can be inhibited.

The boiling point of a fluorinated saturated hydrocarbon and the boiling point of an amine compound referred to the present disclosure can be measured in accordance with JIS K2254 under atmospheric pressure (1 atm).

In the presently disclosed dry etching composition, the amine compound is preferably formed from an amine having a carbon number of at least 3 and not more than 5. An amine having a carbon number of at least 3 and not more than 5 is easy to handle and has an excellent decomposition inhibiting effect with respect to a fluorinated saturated hydrocarbon.

Examples of the fluorinated saturated hydrocarbon contained in the presently disclosed dry etching composition include, but are not specifically limited to, C₃H₇F, C₃H₆F₂, C₄H₉F, C₄H₈F₂, C₅H₁₁F, and C₅H₁₀F₂.

In the presently disclosed dry etching composition, the amine compound preferably has a concentration of at least 0.01 volume% and not more than 1 volume%. Decomposition of the fluorinated saturated hydrocarbon can be sufficiently inhibited when the concentration of the amine compound is at least 0.01 volume% and not more than 1 volume%.

The concentration of an amine compound referred to in the present disclosure can be measured using a gas chromatograph.

A dry etching composition-filled container can be obtained by filling the dry etching composition set forth above into a container having at least an inner surface made from stainless steel, manganese steel, carbon steel, or chromium molybdenum steel.

The maximum roughness depth (Rmax) of the inner surface of the container is preferably 25 µm or less.

### (Advantageous Effect)

Through the presently disclosed dry etching composition, decomposition of a fluorinated saturated hydrocarbon during storage or use can be inhibited.

### DETAILED DESCRIPTION

The following provides a detailed description of embodiments of the present disclosure.

The presently disclosed dry etching composition is a composition that contains a fluorinated saturated hydrocarbon in high concentration and can particularly suitably be used, for example, as a dry etching gas in selective dry etching of a silicon nitride film without any specific limitations.

### (Dry etching composition)

The presently disclosed dry etching composition contains a fluorinated saturated hydrocarbon and an amine compound. The presently disclosed dry etching composition can inhibit decomposition of the fluorinated saturated hydrocarbon during storage or use as a result of containing the amine compound.

Although the reason that decomposition of the fluorinated saturated hydrocarbon can be inhibited is not clear, it is presumed that the amine compound is adsorbed by a Lewis acid present at the surface of a component that the dry etching composition comes into contact with during storage or use thereof, and that this inhibits the occurrence of a decomposition reaction of the fluorinated saturated hydrocarbon (for example, HF-elimination reaction) catalyzed by the Lewis acid, and thereby inhibits decomposition of the fluorinated saturated hydrocarbon. Examples of the component having a Lewis acid at the surface thereof include, but are not specifically limited to, components made from metals such as stainless steel and manganese steel, and components made from solid acids such as aluminum oxide, silicon dioxide, zeolites, and cation exchange resins (proton type).

### <Fluorinated saturated hydrocarbon>

The fluorinated saturated hydrocarbon contained in the presently disclosed dry etching composition is not specifically limited so long as it is a compound having a structure in which a portion of hydrogen atoms of a saturated hydrocarbon are substituted by fluorine atoms, and may be any known fluorinated saturated hydrocarbon that is suitable for use in dry etching.

Although the presently disclosed dry etching composition normally contains only one fluorinated saturated hydrocarbon, the presently disclosed dry etching composition may contain two or more fluorinated saturated hydrocarbons.

More specifically, the fluorinated saturated hydrocarbon is preferably a fluorinated saturated hydrocarbon that has a carbon number of at least 3 and not more than 5. One reason for this is that a fluorinated saturated hydrocarbon having a carbon number of 3 or more benefits from a large decomposition inhibiting effect through the amine compound (i.e., readily undergoes decomposition reaction in the absence of an amine compound). Another reason is that a fluorinated saturated hydrocarbon having a carbon number of 6 or more has a high boiling point and is difficult to use as a dry etching gas.

In particular, fluorinated saturated hydrocarbons such as C₃H₇F, C₃H₆F₂, C₄H₉F, C₄H₈F₂, C₅H₁₁F, and C₅H₁₀F₂ are examples of fluorinated saturated hydrocarbons that benefit from a large decomposition inhibiting effect through the amine compound (i.e., readily undergo decomposition reaction in the absence of an amine compound).

Examples of fluorinated saturated hydrocarbons having a molecular formula C₃H₇F include 1-fluoropropane and 2-fluoropropane, and examples of fluorinated saturated hydrocarbons having a molecular formula C₃H₆F₂ include 1,1-difluoropropane, 1,2-difluoropropane, and 2,2-difluoropropane.

Examples of fluorinated saturated hydrocarbons having a molecular formula C₄H₉F include 1-fluorobutane, 2-fluorobutane, 1-fluoro-2-methylpropane, and 2-fluoro-2-methylpropane, and examples of fluorinated saturated hydrocarbons having a molecular formula C₄H₈F₂ include 1,4-difluorobutane, 2,2-difluorobutane, and 2,3-difluorobutane.

Examples of fluorinated saturated hydrocarbons having a molecular formula C₅H₁₁F include 1-fluoropentane, 2-fluoropentane, 3-fluoropentane, 1-fluoro-2-methylbutane, 1-fluoro-3-methylbutane, 2-fluoro-2-methylbutane, 2-fluoro-3-methylbutane, and 1-fluoro-2,2-dimethylpropane, and examples of fluorinated saturated hydrocarbons having a molecular formula C₅H₁₀F₂ include 1,5-difluoropentane, 2,2-difluoropentane, 3,3-difluoropentane, 2,3-difluoropentane, and 2,4-difluoropentane.

Of the fluorinated saturated hydrocarbons listed above, fluorinated saturated hydrocarbons that do not have a fluorine atom bonded to a molecular terminal carbon atom are examples of fluorinated saturated hydrocarbons that benefit from a particularly large decomposition inhibiting effect through the amine compound. Specific examples of fluorinated saturated hydrocarbons that benefit from a particularly large decomposition inhibiting effect through the amine compound include 2-fluoropropane, 2,2-difluoropropane, 2-fluorobutane, 2-fluoro-2-methylpropane, 2,2-difluorobutane, 2,3-difluorobutane, 2-fluoropentane, 3-fluoropentane, 2-fluoro-2-methylbutane, 2-fluoro-3-methylbutane, 2,2-difluoropentane, 3,3-difluoropentane, 2,3-difluoropentane, and 2,4-difluoropentane.

The concentration of the fluorinated saturated hydrocarbon in the presently disclosed dry etching composition can be adjusted as appropriate so long as it is 99 volume% or more. The concentration of the fluorinated saturated hydrocarbon is preferably 99.50 volume% or more, more preferably 99.80 volume% or more, even more preferably 99.85 volume% or more, and particularly preferably 99.90 volume% or more.

### <Amine compound>

The amine compound may be any amine compound selected from the group consisting of primary amines, secondary amines, and tertiary amines.

Specific examples of the amine compound include methylamine, ethylamine, ethyleneimine, n-propylamine, isopropylamine, cyclopropylamine, azetidine, 1-methylaziridine, n-butylamine, isobutylamine, t-butylamine, n-pentylamine, n-hexylamine, 2-ethylhexylamine, n-nonylamine, n-decylamine, benzylamine, cyclohexylamine, aniline, dimethylamine, diethylamine, ethylmethylamine, di-n-propylamine, diisopropylamine, di-n-butylamine, di-t-butylamine, di-n-pentylamine, trimethylamine, dimethylethylamine, triethylamine, tri-n-propylamine, triisopropylamine, azacyclobutane, pyrrolidine, piperidine, hexamethyleneimine, ethylenediamine, propylenediamine, tetramethylenediamine, N,N,N',N'-tetramethylethylenediamine, pyridine, 2-methylpyridine, 3-methylpyridine, and 4-methylpyridine.

One amine compound may be used individually, or two or more amine compounds may be used in combination.

Of these amine compounds, amine compounds having a carbon number of at least 3 and not more than 5 are preferable, and amine compounds having a carbon number of 3 or 4 are more preferable. One reason for this is that amine compounds having a carbon number of at least 3 and not more than 5 are easy to handle. Another reason is that amine compounds having a carbon number of 5 or less are readily adsorbed by Lewis acids and the like, and have an excellent decomposition inhibiting effect with respect to fluorinated saturated hydrocarbons.

Moreover, from a viewpoint of ease of handling, the amine compound is preferably a liquid under normal temperature and normal pressure. Specifically, the melting point of the amine compound under atmospheric pressure (1 atm) is preferably 10°C or lower, and more preferably 0°C or lower. Moreover, the boiling point of the amine compound under atmospheric pressure (1 atm) is preferably 25°C or higher, and more preferably 30°C or higher, and is preferably 50°C or lower, and more preferably 40°C or lower.

The absolute value of the difference between the boiling point of the fluorinated saturated hydrocarbon described above and the boiling point of the amine compound is preferably 25°C or less, more preferably 20°C or less, and even more preferably 10°C or less. This is because when the absolute value of the difference between the boiling point of the fluorinated saturated hydrocarbon and the boiling point of the amine compound is small, the fluorinated saturated hydrocarbon and the amine compound are both favorably vaporized at the temperature of use of the dry etching composition, and thus variation of the make-up of the dry etching composition during use can be inhibited.

The concentration of the amine compound in the presently disclosed dry etching composition is normally 1 volume% or less, preferably at least 0.01 volume% and not more than 1 volume%, more preferably at least 0.01 volume% and not more than 0.15 volume%, and even more preferably at least 0.01 volume% and not more than 0.1 volume%. One reason for this is that decomposition of the fluorinated saturated hydrocarbon can be sufficiently inhibited when the concentration of the amine compound is 0.01 volume% or more. Another reason is that the fluorinated saturated hydrocarbon can be contained in the dry etching composition in a sufficiently high concentration while sufficiently inhibiting decomposition of the fluorinated saturated hydrocarbon when the concentration of the amine compound is 1 volume% or less.

### <Properties of dry etching composition>

The presently disclosed dry etching composition is preferably an azeotropic composition or an azeotrope-like composition. This is because variation of the make-up of the dry etching composition during use when the dry etching composition is vaporized for use in dry etching can be inhibited in a situation in which the dry etching composition is an azeotropic composition or an azeotrope-like composition.

The dry etching composition can be prepared as an azeotropic composition or an azeotrope-like composition by appropriately selecting a combination of the types of components contained in the composition, the concentrations thereof, and so forth.

### <Production method>

The presently disclosed dry etching composition can be produced by mixing the fluorinated saturated hydrocarbon and amine compound set forth above by a known method without any specific limitations.

Note that although components other than the fluorinated saturated hydrocarbon and the amine compound can also be compounded in the presently disclosed dry etching composition, it is preferable that the presently disclosed dry etching composition is produced through mixing of only the fluorinated saturated hydrocarbon and the amine compound. In other words, the presently disclosed dry etching composition preferably only contains the fluorinated saturated hydrocarbon, the amine compound, and impurities that are unavoidably mixed in during production of the dry etching composition.

The presently disclosed dry etching composition that is produced may, for example, be filled into and stored in a container or the like having at least an inner surface made from a metal (for example, stainless steel, manganese steel, carbon steel, or chromium molybdenum steel) without any specific limitations. In such a situation, decomposition of the fluorinated saturated hydrocarbon during storage is inhibited as a result of the presently disclosed dry etching composition containing the amine compound.

The container having at least an inner surface made from a metal may be any container in which at least an inner surface part thereof is made from a metal such as stainless steel, manganese steel, carbon steel, or chromium molybdenum steel, and is not required to be a container that is entirely made from a metal. The inner surface of the container having at least an inner surface made from a metal may be subjected to polishing by barrel polishing or the like. Moreover, the maximum roughness depth (Rmax) of the inner surface of the container is preferably 25 µm or less, and more preferably 5 µm or less. Although the maximum roughness depth (Rmax) of the inner surface of the container does not have a specific lower limit, the maximum roughness depth (Rmax) is normally 1 µm or more. The maximum roughness depth of an inner surface of a container can be measured by a surface roughness measurement device.

### EXAMPLES

The following provides a more specific description of the present disclosure based on examples. However, the present disclosure is not limited to the following examples. In the following description, "%" and "parts" used in expressing quantities are by mass, unless otherwise specified.

The following methods were used in the examples and comparative examples to measure and evaluate the concentrations of fluorinated saturated hydrocarbons, amine compounds, and other compounds, and the stability of dry etching compositions.

### <Concentrations of fluorinated saturated hydrocarbons, amine compounds, and other compounds>

The concentrations of fluorinated saturated hydrocarbons, the concentrations of amine compounds, and the concentrations of other compounds were measured for produced dry etching compositions by gas chromatography.

The apparatus (gas chromatograph) and conditions used in concentration measurement were as follows.
- Apparatus: Agilent® 7890A (Agilent is a registered trademark in Japan, other countries, or both) produced by Agilent Technologies
- Column: Produced by GL Sciences Inc.; product name: InertCap® 1 (InertCap is a registered trademark in Japan, other countries, or both); length: 60 m; internal diameter: 0.25 mm; film thickness: 1.5 µm
- Column temperature: Held at 40°C for 20 minutes
- Injection temperature: 80°C
- Carrier Gas: Nitrogen
- Split ratio: 40/l
- Detector: FID

### <Stability of dry etching compositions>

The stability of each dry etching composition was evaluated using a stability evaluation apparatus in which a composition filling container and a gas chromatograph were linked by piping made from SUS-316 that was equipped with a filter made from alumina ceramic (produced by PURERON JAPAN Co., Ltd.; model: PDF-3-02SW·PC07) and a mass flow controller (produced by HORIBA STEC Co., Ltd.; model: SEC-2511X).

Specifically, the produced dry etching composition was filled into the composition filling container and was subsequently caused to flow from the composition filling container to the gas chromatograph while the filter was heated to 50°C by a heater. The concentration of fluorinated saturated hydrocarbon in dry etching composition that passed through the filter and flowed into the gas chromatograph was measured. The concentration (C₀) of fluorinated saturated hydrocarbon in the produced dry etching composition and the concentration (C₁) of fluorinated saturated hydrocarbon in the dry etching composition that flowed into the gas chromatograph were compared to confirm whether fluorinated saturated hydrocarbon decomposition had occurred.

Note that a container made from manganese steel (inner surface Rmax: 25 µm or less) was used as the composition filling container. The gas chromatograph and conditions used in measurement of the concentration (C₁) of fluorinated saturated hydrocarbon were as follows.
- Gas chromatograph: Agilent® 7890A produced by Agilent Technologies
- Column: Produced by GL Sciences Inc.; product name: InertCap® 1; length: 60 m; internal diameter: 0.25 mm; film thickness: 1.5 µm
- Column temperature: Held at 40°C for 20 minutes
- Injection temperature: 80°C
- Carrier gas: Nitrogen
- Split ratio: 40/l
- Detector: FID

### (Examples 1 to 3)

A dry etching composition (containing unavoidably mixed in impurities) was produced by mixing 2-fluorobutane as a fluorinated saturated hydrocarbon and an amine compound shown in Table 1. The concentrations of compounds contained in the dry etching composition were measured and the stability of the dry etching composition was evaluated. The results are shown in Table 1.

### (Comparative Example 1)

Measurement and evaluation were carried out in the same way as in Example 1 with the exception that a dry etching composition containing only 2-fluorobutane and unavoidably mixed in impurities was used without using an amine compound. The results are shown in Table 1.

### (Comparative Examples 2 and 3)

A dry etching composition (containing unavoidably mixed in impurities) was produced by mixing 2-fluorobutane as a fluorinated saturated hydrocarbon and another compound shown in Table 1 without using an amine compound. The concentrations of compounds contained in the dry etching composition were measured and the stability of the dry etching composition was evaluated. The results are shown in Table 1.

**Table 1**

| | | | Example 1 | Example 2 | Example 3 | Comparative Example 1 | Comparative Example 2 | Comparative Example 3 |
|---|---|---|---|---|---|---|---|---|
| Dry etching composition | Fluorinated saturated hydrocarbon | 2-Fluorobutane (boiling point: 25°C) [volume%] | 99.86 | 99.90 | 99.86 | 99.96 | 99.85 | 99.88 |
| | Amine compound | Isopropylamine (melting point: -9S.2°C, boiling point: 34°C) [volume%] | 0.13 | - | - | - | - | - |
| | | Ethylmethylamine (melting point: -30°C, boiling point: 37°C) [volume%] | - | 0.09 | - | - | - | - |
| | | Dimethylethylamine (melting point: -140°C, boiling point: 36.5°C) [volume%] | - | - | 0.13 | - | - | - |
| | Other compound | Chloroform (melting point: -64°C, boiling point: 61.2°C) [volume%] | - | - | - | - | 0.12 | - |
| | | Methanol (melting point: -97°C, boiling point: 64.7°C) [volume%] | - | - | - | - | - | 0.11 |
| Evaluation | Stability (occurrence of decomposition) | | No | No | No | Yes | Yes | Yes |

### (Example 4)

A dry etching composition (containing unavoidably mixed in impurities) was produced by mixing 2,2-difluorobutane as a fluorinated saturated hydrocarbon and an amine compound shown in Table 2. The concentrations of compounds contained in the dry etching composition were measured and the stability of the dry etching composition was evaluated. The results are shown in Table 2.

### (Comparative Example 4)

Measurement and evaluation were carried out in the same way as in Example 4 with the exception that a dry etching composition containing only 2,2-difluorobutane and unavoidably mixed in impurities was used without using an amine compound. The results are shown in Table 2.

### (Comparative Example 5)

A dry etching composition (containing unavoidably mixed in impurities) was produced by mixing 2,2-difluorobutane as a fluorinated saturated hydrocarbon and another compound shown in Table 2 without using an amine compound. The concentrations of compounds contained in the dry etching composition were measured and the stability of the dry etching composition was evaluated. The results are shown in Table 2.

**Table 2**

| | | | Example 4 | Comparative Example 4 | Comparative Example 5 |
|---|---|---|---|---|---|
| Dry etching composition | Fluorinated saturated hydrocarbon | 2,2-Difluorobutane (boiling point: 32°C) [volume%] | 99.59 | 99.73 | 99.60 |
| | Amine compound | Isopropylamine (melting point: -95.2°C, boiling point: 34°C) [volume%] | 0.13 | - | - |
| | Other compound | Methanol (melting point: -97°C, boiling point: 64.7°C) [volume%] | - | - | 0.12 |
| Evaluation | Stability (occurrence of decomposition) | | No | Yes | Yes |

### (Examples 5 and 6)

A dry etching composition (containing unavoidably mixed in impurities) was produced by mixing 1-fluorobutane as a fluorinated saturated hydrocarbon and an amine compound shown in Table 3. The concentrations of compounds contained in the dry etching composition were measured and the stability of the dry etching composition was evaluated. The results are shown in Table 3.

### (Comparative Example 6)

Measurement and evaluation were carried out in the same way as in Example 5 with the exception that a dry etching composition containing only 1-fluorobutane and unavoidably mixed in impurities was used without using an amine compound. The results are shown in Table 3.

**Table 3**

| | | | Example 5 | Example 6 | Comparative Example 6 |
|---|---|---|---|---|---|
| Dry etching composition | Fluorinated saturated hydrocarbon | 1-Fluorobutane (boiling point: 32°C) [volume%] | 99.60 | 99.61 | 99.72 |
| | Amine compound | Isopropylamine (melting point: -95.2°C, boiling point: 34°C) [volume%] | 0.12 | - | - |
| | | Dimethylethylamine (melting point: -140°C, boiling point: 36.5°C) [volume%] | - | 0.11 | - |
| Evaluation | Stability (occurrence of decomposition) | | No | No | Yes |

### (Example 7)

A dry etching composition (containing unavoidably mixed in impurities) was produced by mixing 2-fluoropentane as a fluorinated saturated hydrocarbon and an amine compound shown in Table 4. The concentrations of compounds contained in the dry etching composition were measured and the stability of the dry etching composition was evaluated. The results are shown in Table 4.

### (Comparative Example 7)

Measurement and evaluation were carried out in the same way as in Example 7 with the exception that a dry etching composition containing only 2-fluoropentane and unavoidably mixed in impurities was used without using an amine compound. The results are shown in Table 4.

**Table 4**

| | | | Example 7 | Comparative Example 7 |
|---|---|---|---|---|
| Dry etching composition | Fluorinated saturated hydrocarbon | 2-Fluoropentane (boiling point: 57°C) [volume%] | 99.70 | 99.83 |
| | Amine compound | Dimethylethylamine (melting point: -140°C, boiling point: 36.5°C) [volume%] | 0.13 | - |
| Evaluation | Stability (occurrence of decomposition) | | No | Yes |

### (Example 8)

A dry etching composition (containing unavoidably mixed in impurities) was produced by mixing 2-fluoropropane as a fluorinated saturated hydrocarbon and an amine compound shown in Table 5. The concentrations of compounds contained in the dry etching composition were measured and the stability of the dry etching composition was evaluated. The results are shown in Table 5.

### (Comparative Example 8)

Measurement and evaluation were carried out in the same way as in Example 8 with the exception that a dry etching composition containing only 2-fluoropropane and unavoidably mixed in impurities was used without using an amine compound. The results are shown in Table 5.

**Table 5**

| | | | Example 8 | Comparative Example 8 |
|---|---|---|---|---|
| Dry etching composition | Fluorinated saturated hydrocarbon | 2-Fluoropropane (boiling point: -9.5°C) [volume%] | 99.40 | 99.53 |
| | Amine compound | Methylamine (melting point: -93°C, boiling point: -6°C) [volume%] | 0.13 | - |
| Evaluation | Stability (occurrence of decomposition) | | No | Yes |

It can be seen from Tables 1 to 5 that fluorinated saturated hydrocarbon decomposition was inhibited in the dry etching compositions of Examples 1 to 8 in which an amine compound was compounded.

### (Examples 9 to 18 and Comparative Examples 9 to 14)

A dry etching composition (containing unavoidably mixed in impurities) having the make-up shown in Table 6 was produced. In addition, a gas filling container (inner surface Rmax: 25 µm or less) made from a material shown in Table 6 was prepared.

Next, the prepared gas filling container was filled with the dry etching composition and was left at rest for 30 days at a temperature of 55°C. The post-resting concentration of fluorinated saturated hydrocarbon was measured by a gas chromatograph, and the amount of decomposition of fluorinated saturated hydrocarbon was calculated. The results are shown in Table 6.

The gas chromatograph and conditions used in measurement of the fluorinated saturated hydrocarbon concentration were as follows.
- Gas chromatograph: Agilent® 7890A produced by Agilent Technologies
- Column: Produced by GL Sciences Inc.; product name: InertCap® 1; length: 60 m; internal diameter: 0.25 mm; film thickness: 1.5 µm
- Column temperature: Held at 40°C for 20 minutes
- Injection temperature: 80°C
- Carrier gas: Nitrogen
- Split ratio: 40/l
- Detector: FID

In Table 6, shown below:
"SUS" indicates stainless steel;
"Mn" indicates manganese steel; and
"CrMo" indicates chromium molybdenum steel.

**Table 6**

| | | | Example 9 | Example 10 | Example 11 | Example 12 | Example 13 | Comparative Example 9 | Comparative Example 10 | Example 14 | Comparative Example 11 | Example 15 | Example 16 | Comparative Example 12 | Example 17 | Comparative Example 13 | Example 18 | Comparative Example 14 |
|---|---|---|---|---|---|---|---|---|---|---|---|---|---|---|---|---|---|---|
| | | 2-Fluorobutane (boiling point: 25°C) [volume%] | 99.86 | 99.86 | 99.86 | 99.90 | 99.86 | 99.95 | 99.88 | - | - | - | - | - | - | - | - | - |
| | | (2,2-Difluorobutane (boiling point: 32°C) [volume%] | - | - | - | - | - | - | - | 99.59 | 99.73 | - | - | - | - | - | - | - |
| | Fluorinated saturated hydrocarbon | 1-Fluorobutane (boiling point: 32°C) [volume%] | - | - | - | - | - | - | - | - | - | 99.60 | 99.61 | 99.72 | - | - | - | - |
| | | 2-Fluoropentane (boiling point: 57°C) [volume%] | - | - | - | - | - | - | - | - | - | - | - | - | 99.70 | 99.83 | - | - |
| | | 2-Fluoropropane (boiling point: -9.5°C) [volume%] | - | - | - | - | - | - | - | - | - | - | - | - | - | - | 99.40 | 99.53 |
| Dry etching composition | | Isopropylamine (melting point: -95.2°C, boiling point: 34°C) [volume%] | 0.13 | 0.13 | 0.13 | - | - | - | - | 0.13 | - | 0.12 | - | - | - | - | - | - |
| | | Ethylmethylamine (melting point: -30°C, boiling point: 37°C) [volume%] | - | - | - | 0.09 | - | - | - | - | - | - | - | - | - | - | - | - |
| | Amine compound | Dimethylethylamine (melting point: -140°C, boiling point: 36.5°C) [volume%] | - | - | - | - | 0.13 | - | - | - | - | - | 0.11 | - | 0.13 | - | - | - |
| | | Methylamine (melting point: -93°C, boiling point: -6°C) [volume%] | - | - | - | - | - | - | - | - | - | - | - | - | - | - | 0.13 | - |
| | Other compound | Methanol (melting point: -97°C, boiling point: 64.7°C) [volume%] | - | - | - | - | - | - | 0.11 | - | - | - | - | - | - | - | - | - |
| Gas filling container | Material | | SUS | Mn | CrMo | SUS | SUS | SUS | SUS | SUS | SUS | SUS | SUS | SUS | SUS | SUS | SUS | SUS |
| Evaluation | Concentration of fluorinated saturated hydrocarbon after 30 days [volume%] | | 99.86 | 99.86 | 99.86 | 99.90 | 99.86 | 98.97 | 99.68 | 99.59 | 99.43 | 99.60 | 99.61 | 98.90 | 99.70 | 98.80 | 99.40 | 99.10 |
| | Amount of decomposition of fluorinated saturated hydrocarbon [volume%] | | 0 | 0 | 0 | 0 | 0 | 0.98 | 0.20 | 0 | 0.30 | 0 | 0 | 0.82 | 0 | 1.03 | 0 | 0.43 |

It can be seen from Table 6 that fluorinated saturated hydrocarbon decomposition was inhibited in the dry etching compositions of Examples 9 to 18 in which an amine compound was compounded.

### INDUSTRIAL APPLICABILITY

Through the presently disclosed dry etching composition, decomposition of a fluorinated saturated hydrocarbon during storage or use can be inhibited.

## Claims

1. A dry etching composition comprising:
99 volume% or more of a fluorinated saturated hydrocarbon; and
an amine compound.

2. The dry etching composition according to claim 1, wherein
the dry etching composition is an azeotropic composition or an azeotrope-like composition.

3. The dry etching composition according to claim 1 or 2, wherein
an absolute value of a difference between a boiling point of the fluorinated saturated hydrocarbon and a boiling point of the amine compound is 25°C or less.

4. The dry etching composition according to any one of claims 1 to 3, wherein
the amine compound is formed from an amine having a carbon number of at least 3 and not more than 5.

5. The dry etching composition according to any one of claims 1 to 4, wherein
the fluorinated saturated hydrocarbon is C₃H₇F, C₃H₆F₂, C₄H₉F, C₄H₈F₂, C₅H₁₁F, or C₅H₁₀F₂.

6. The dry etching composition according to any one of claims 1 to 5, wherein
the amine compound has a concentration of at least 0.01 volume% and not more than 1 volume%.

7. A dry etching composition-filled container comprising a container having at least an inner surface made from stainless steel, manganese steel, carbon steel, or chromium molybdenum steel and filled with the dry etching composition according to any one of claims 1 to 6.

8. The dry etching composition-filled container according to claim 7, wherein
the inner surface of the container has a maximum roughness depth (Rmax) of 25 µm or less.
